# EUROPÄISCHE PATENTANMELDUNG

(11) **EP 0 887 083 A1**
(43) Veröffentlichungstag der Anmeldung: **30.12.1998**
(21) Anmeldenummer: 98111232.9
(22) Anmeldetag: 18.06.1998
(51) Int. Cl.: A61M 5/42, A61M 37/00, A61K 39/00, A61D 1/02

(54) **Gerät zum Injizieren eines Materials, insbesondere eines Impfstoffes, in eine Ohrmuschel**

(30) Priorität: 23.06.1997 DE 19726599
(71) Anmelder: Schirrmacher, Roland, Dipl.-Ing. FH, 82166 Gräfelfing (DE)
(72) Erfinder: Schirrmacher, Roland, 82166 Gräfelfing (DE)
(74) Vertreter: Blumbach, Kramer & Partner GbR

(57) **Zusammenfassung**

Ein Impfverfahren zeichnet sich dadurch aus, daß der vorzugsweise DNA und/oder RNA enthaltende Impfstoff in eine Ohrmuschel injiziert wird. Ein Gerät zum Injizieren eines Materials, insbesondere eines Impfstoffes, in eine Ohrmuschel enthält eine Klemmvorrichtung (6, 14) zum Einklemmen eines Bereiches der Ohrmuschel und eine Injektionsvorrichtung (36, 46, 48), die bei in der Klemmvorrichtung eingeklemmten Ohrbereich auf das Ohr zu bewegbar ist und zum Injizieren des Materials in die Ohrmuschel eindringt.

## Beschreibung

Die Erfindung betrifft ein Impfverfahren, Verwendung eines Impfstoffs und Gerät zum Injizieren eines Materials, insbesondere eines Impfstoffes in eine Ohrmuschel.

Impfungen werden in der Regel durch Applikation einer Vakzine in Muskelgewebe (intramusculär, i.m.) oder in die Haut (interdermal, i.d.) oder unter die Haut (subcutan, s.c.) vollzogen. Eine Neuentwicklung auf dem Gebiet der Vakzine-Forschung, die seit einigen Jahren verfolgt wird, besteht in dem direkten Gentransfer in Form von Fremd-DNA oder RNA (Ribonukleinsäuren). Ein Vorteil bei dieser genetischen Immunisierung besteht in der einfachen Präparation von DNA in großer Reinheit und Menge. Außerdem ist DNA im Vergleich zu Proteinen sehr stabil und erlaubt unkomplizierte Lagerung, Transport und Handhabung.

Die ersten genetischen Immunisierungen erfolgten intramasculär. Erst später kam die Hautimmunisierung hinzu. Während die intramusculäre Applikation von DNA mit einer Kanüle erfolgen kann, wird zur Transfektion der Haut mit DNA immer häufiger die Gene-Gun (Genpistole) als Methode angewendet. Hiebei müssen jedoch Goldpartikel verwendet werden, die mit DNA beschichtet werden, was teuer und arbeitsintensiv ist und deshalb keinen wesentlichen Vorteil darstellt.

Hautgewebe unterscheidet sich von Muskelgewebe unter anderem dadurch, daß es reichhaltig mit Zellen der körpereigenen Abwehr durchsetzt ist. Die Haut stellt nicht nur eine anatomische Barriere gegen eindringende Keime dar, sondern auch eine immunologische Barriere. In die Haut eindringende Mikroorganismen mit ihren verschiedenen Fremdstrukturen inklusive Eiweiß und DNA werden hier von spezialisierten Zellen angegriffen, abgebaut und mit dem lymphatischen Abfluß in dränierende Lymphknoten transportiert, wo es zur Induktion von spezifischen Immunabwehrreaktionen kommt.

Der Erfindung liegt die Aufgabe zugrunde, die Wirksamkeit von Impfungen zu verbessern. Der Erfindung liegt weiter die Aufgabe zugrunde, ein in seinem Aufbau einfaches Gerät zu schaffen, mit dem eine besonders wirksame Injektion von Material, insbesondere eines Impfstoffs, möglich ist.

Der erste Teil der Aufgabe wird mit den Merkmalen des Anspruchs 1 gelöst.

Die Ohrmuschel verkörpert eine Sonderform der Haut. Sie besitzt zwei spiegelbildlich angeordnete Hautschichten, die von einem dazwischenliegenden Knorpel getrennt werden. Die geringe Behaarung und die exponierte Position legen nahe, daß eine schnelle und effektive immunologische Reaktion auf eindringende Keime notwendig ist. Es ist schon länger bekannt, daß das Ohr ein potenter Ort zur Induktion zellulärer Immunreaktionen ist. Dieses könnte mit einer weiteren anatomischen Besonderheit zu tun haben, nämlich daß das Ohr überwiegend von einem Lymphknoten (Superfiszcia cervicales) dräniert wird, was eine Konzentrierung der Immunantwort auf einen Lymphknoten gewährleistet, während andere Hautstellen in der Regel von mehreren Lymphknoten dräniert werden.

Mit dem erfindungsgemäßen Impfverfähren wird somit die Wirksamkeit des Impfstoffes und damit die Wirkung der Impfung überhaupt verbessert.

Besonders vorteilhaft ist das erfindungsgemäße Verfahren für Impfstoffe gemäß dem Anspruch 2.

Der Anspruch 3 kennzeichnet eine weitere Lösung der Aufgabe, die Wirksamkeit eines Impfstoffes zu verbessern. Dies wird dadurch erreicht, daß einem Stoff, der an sich bekannt ist, in eine Ohrmuschel injiziert wird.

Vorteilhafterweise enthält der Impfstoff DNA und/oder RNA.

Der Anspruch 5 ist auf die grundsätzliche Konstruktion eines Gerätes gerichtet, mit dem der zweite Teil der Erfindungsaufgabe gelöst wird. Mit dem erfindungsgemäßen Gerät, das auch als Ohr-Stempel bezeichnet werden könnte, können unterschiedlichste Materialien in die Haut der Ohrmuschel injiziert werden. Unter Ohrmuschel wird dabei das gesamte Außenohr einschließlich des Ohrläppchens verstanden. Die Applikationsstelle kann sowohl die Außenseite als auch die Innenseite der Ohrmuschel sein. Das Gerät ist vorteilhafterweise als Handgerät konzipiert, welches von Links- und Rechtshändern gleich gut bedient werden kann, unabhängig davon, ob das linke oder das rechte Ohr benutzt wird. Das Gerät ist als Einhandgerät konzipiert, so daß die zweite Hand für andere Tätigkeiten frei ist, beispielsweise das Weghalten von Haaren hinter dem Ohr.

Das erfindungsgemäße Gerät zeichnet sich dadurch aus, daß die Ohrmuschel mittels der Klemmvorrichtung gehalten wird und die Injektionsvorrichtung in definierter Weise zum Injizieren des Materials, insbesondere des Impfstoffes, in die Ohrmuschel eindringt. Dabei sind die unterschiedlichsten Konstruktionen der Klemmvorrichtung und Injektionsvorrichtung möglich, beispielsweise selbstschließende Klemmvorrichtungen, die von Hand offen gehalten werden müssen, oder Klemmvorrichtungen, die von Hand geschlossen werden müssen. Die Injektionsvorrichtung kann ebenfalls unterschiedlichster Bauart sein, beispielsweise ein Stempel mit einer oder mehreren Spitzen oder Nadeln oder Röhrchen.

Bei der Ausführungsform des Gerätes gemäß dem Anspruch 6 ist die Injektionsvorrichtung starr an einem Teil der Klemmvorrichtung befestigt. Diese Konstruktion ist besonders einfach, erfordert jedoch eine sorgfältige Handhabung, da die Injektionsspritzen mit dem Ohr bereits in Berührung kommen können, bevor das Ohr eingeklemmt ist.

Mit den Mermalen des Anspruchs 7 wird der Vorteil erreicht, daß die Injektionsvorrichtung bzw. deren Spritzen oder Nadeln erst dann auf das Ohr zu bewegt wird bzw. die Ohrhaut durchsticht, wenn die Ohrmuschel eingeklemmt und damit "ortsfest" gehalten ist. Die Injektionsvorrichtung kann unterschiedlichster Bauart sein und auf unterschiedliche Weise an der Klemmvorrichtung beweglich gehalten sein, beispielsweise durch eine lineare Führung, durch eine Anlenkung usw..

Der Anspruch 8 ist auf eine vorteilhafte Ausführungsform des Gerätes nach Anspruch 7 gerichtet, das bezüglich seiner Handhabbarkeit besonders einfach ist.

Mit den Merkmalen des Anspruchs 9 wird erreicht, daß der das zu injizierende Material tragende Vorsprung sich plötzlich um eine vorbestimmte Strecke in Richtung auf das Ohr bewegt, so daß bei Ausbildung des Vorsprungs in Form von Nadelspitzen oder als Kanüle diese sicher in die Ohrmuschel eindringen und das Material injiziert wird.

Die Ansprüche 10 und 11 kennzeichnen unterschiedliche vorteilhafte Ausführungsformen des Materialträgers.

Mit den Merkmalen des Anspruchs 12 wird erreicht, daß eine wohldefinierte Menge an Flüssigkeit aus der Kanüle in die Ohrmuschel eingespritzt wird.

Mit den Merkmalen des Anspruchs 13 läßt sich die Eindringtiefe der Nadeln bzw. der Kanüle in die Ohrmuschel einstellen.

Mit den Merkmalen des Anspruchs 14 wird erreicht, daß der Materialträger in einfacher Weise auswechselbar ist.

Der Anspruch 15 kennzeichnet ein vorteilhaftes Konstruktionsmerkmal des Gerätes.

Mit den Merkmalen des Anspruchs 16 wird eine besonders komfortable Bedienbarkeit erreicht.

Die Ansprüche 17 und 18 schließlich sind auf eine Ausführungsform des Gerätes gerichtet, die sich als Wegwerfgerät zum einmaligen Gebrauch eignet.

Die Erfindung wird im folgenden anhand schematischer Zeichnungen beispielsweise und mit weiteren Einzelheiten erläutert.

Es stellen dar:
- Fig. 1: einen Längsschnitt durch eine erste Ausführungsform des Gerätes, mit einem Zangenteil in drei unterschiedlichen Stellungen;
- Fig. 2: eine Aufsicht auf das Gerät gemäß Fig. 1 ohne Zangenteil und teilweise aufgeschnitten;
- Fig. 3: eine Ansicht des Gerätes gemäß Fig. 1 von der Bestückungsseite her;
- Fig.4: einen Längsschnitt durch eine gegenüber Fig. 1 abgeänderte Ausführungsform des Gerätes;
- Fig. 5: ein Federdiagramm;
- Fig. 6: einen Teillängsschnitt durch die Ausführungsform gemäß Fig. 1 mit abgeändertem Materialträger;
- Fig. 7: einen Detailschnitt durch den Materialträger der Fig. 6;
- Fig. 8: einen Längsschnitt durch ein Gerät für Einmalbenutzung;
- Fig. 9: eine Aufsicht auf das Gerät gemäß Fig. 8; und
- Fig. 10: einen Längsschnitt durch eine weitere Ausführungsform des Gerätes.

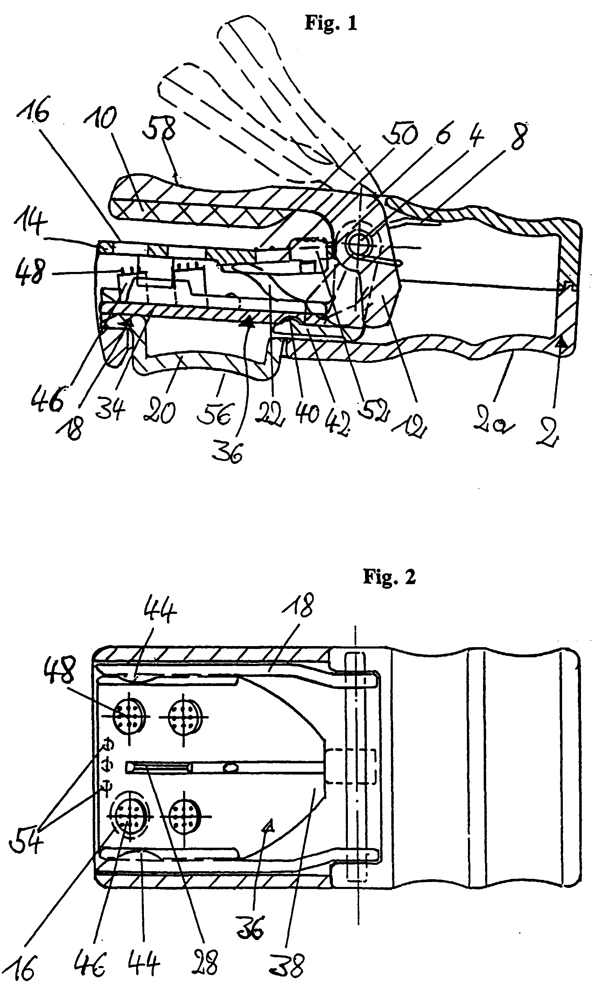

Gemäß Fig. 1 bis 3 ist an einem Gehäuse 2 mittels eines gehäusefesten Bolzens 4 ein Zangenteil 6 gelagert und mittels einer Schenkelfeder 8 in Uhrzeigerrichtung elastisch vorgespannt. An der Innenseite des Zangenteils 6 ist ein Kissen 10 aus weichem Material, beispielsweise Polymer, angeordnet. Jenseits des Bolzens 4 endet das Zangenteil 6 in einem Auswerfernocken 12, dessen Funktion weiter unten erläutert wird.

Einteilig mit dem Gehäuse 2 ausgebildet ist ein Anlageteil 14, das Aussparungen 16 aufweist.

Ebenfalls an dem Bolzen 4 schwenkbar gelagert ist ein Aufnahmeteil 18, welches mit einer durch ein Unterteil 2a des Gehäuses 2 vorstehenden Taste 20 ausgebildet ist. Das Aufnahmeteil 18 wird von einer Feder 22 in Gegenuhrzeigerrichtung vorgespannt, so daß die Taste 20 aus dem Gehäuseunterteil 2a vorsteht.

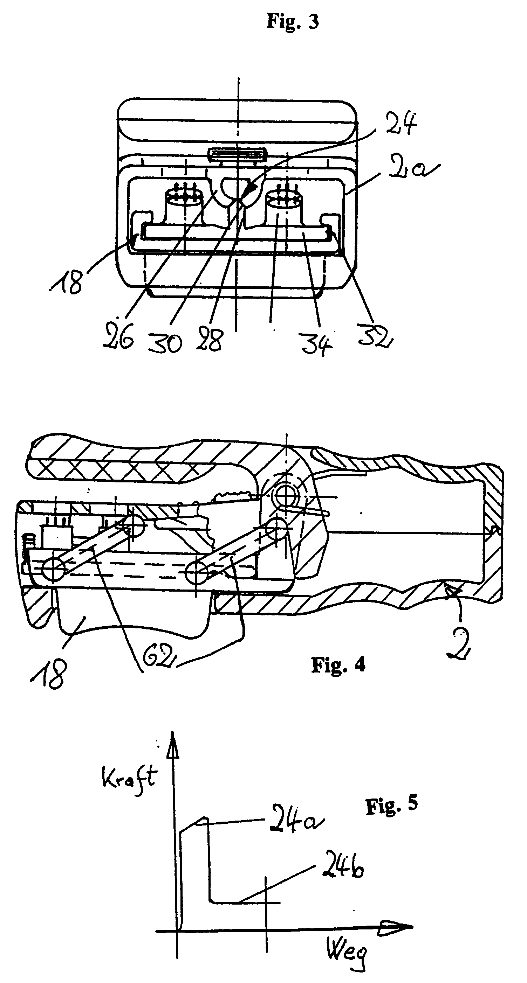

Weiter ist zwischen dem Aufnahmeteil 18 und dem Anlageteil 14 eine Federeinrichtung 24 wirksam, deren Kraft zunächst stark zunimmt und dann plötzlich abfällt. Diese Federeinrichtung 24 ist durch zwei halbkreisförmige Kunststoffedern 26 gebildet, die an dem Anlageteil 14 ausgebildet sind, und einem Steg 28, der an dem Aufnahmeteil 18 ausgebildet ist und sich mit seinen Keilflanken 30 zwischen die Kunststoffedern 26 schiebt. Solange die Keilflanken 30 in Eingriff sind, steigt die Kraft (Fig. 5) bis zum Wert 24a, um dann auf den Wert 24b abzufallen. Der Anschlag des vorderen Endes des Steges 28 an die Grundfläche zwischen den Federn 26 kann eine Bewegungsbegrenzung bilden.

Das Aufnahmeteil 18 ist mit nach innen weisenden, seitlichen Einschubkanälen 32 ausgebildet, in die eine Tragplatte 34 eines Materialträgers 36 einschiebbar ist. Die Tragplatte ist an ihrem vorderen Ende 38 mit einer Rastausnehmung 40 versehen, die im eingeschobenen Zustand der Tragplatte 34 in eine Rastfeder 42 einrastet, die einteilig mit dem Aufnahmeteil 18 ausgebildet ist. Der Auswerfernocken 12 des Zangenteils 6 kommt beim Öffnen des Zangenteils (mittlere eingezeichnete Stellung) in Berührung mit dem vorderen Ende 38 der Tragplatte 34, so daß diese bei weiterem Öffnen des Zangenteils 6 aus dem Aufnahmeteil 18 ausgeschoben wird. Zur besseren Handhabbarkeit weist die Tragplatte 34 seitliche Griffmulden 44 auf.

Die Tragplatte 34 trägt ein bis vier vorspringende Stempelköpfe 46, die auf ihrer Oberseite Nadelspitzen 48 aufweisen. Die Nadelspitzen 48 werden vor der Benutzung mit dem zu applizierenden Material beschickt. Entweder geschieht dies im Labor oder der gesamte Materialträger 36 wird fertig bezogen. In diesem Fall sind die Stempelköpfe 46 mit nicht dargestellten Kappen versehen, die vor Benutzung abgenommen werden.

Als Tiefenanschlag dient der verlängerte Steg 28, der anstatt am Aufnahmeteil 18 am Materialträger 36 ausgebildet sein kann. Für einen Anschlag des Stegs 28 ist auf einer schiefen Ebene 50 im Gehäuse 2 ein Anschlagschieber 52 gelagert, der sich beispielsweise in drei Positionen arretieren läßt und auf diese Weise drei unterschiedlich große Wege für den Materialträger 36 erlaubt.

Eine zweite Version für die Tiefeneinstellung kann wie folgt sein: Der Anschlagschieber 52 entfällt. Auf der Tragplatte 34 des Materialträgers 36 sind drei zylinderförmige Anschläge 54 mit unterschiedlichen Höhen ausgebildet. Bei erwünschtem tieferen Eindringen in die Ohrmuschel wird der jeweils längere Zylinder weggebrochen. Es ist auch denkbar, Stempelträger für unterschiedliche Eindringtiefen, d.h. mit jeweils unterschiedlich langem Anschlag, in unterschiedlichen Farben zu fertigen.

Die Funktion des beschriebenen Gerätes ist folgende:

Der Zusammenbau geschieht derart, daß das Zangenteil 6 und das Aufnahmeteil 18 bei abgenommenem Gehäuseunterteil 2a an dem Gehäuse 2 montiert werden, wobei das Gehäuseunterteil 2a dann aufgesteckt wird. Anschließend wird das Aufnahmeteil 18 mit dem Materialträger 36 bestückt und das Gerät ist betriebsbereit.

Das Gerät wird nun in eine Hand genommen, wobei beispielsweise der Daumen in eine Griffmulde 56 der Taste 20 eingreift und der Zeigefinger in eine Griffmulde 58 des Zangenteils 6 eingreift. Das Gerät wird nun derart an einer Ohrmuschel angesetzt, daß diese zwischen dem Zangenteil 6 und dem Anlageteil 14 angeordnet ist. Durch Schließen der Hand wird das Zangenteil 6 geschlossen, so daß die Ohrmuschel zwischen dem Kissen 10 des Zangenteils 6 und dem Anlageteil 14 unter Flachdrücken gehalten wird, so daß, unabhängig davon, ob die Außen- oder Innenseite der Ohrmuschel an dem Anlageteil 14 anliegt, eine gute Anlage gewährleistet ist. Das Zangenteil 6 schließt zuerst, da die Kraft der Schenkelfeder 8 geringer ist als die der Feder 22 und zusätzlich der Federeinrichtung 24. Wird die Schließkraft der Hand nun weiter erhöht, so tritt unter Überwindung der Kraft der Feder 22 die Federeinrichtung 24 in Funktion, so daß nach Erhöhung der Kraft bis zum Wert 24a (Fig. 5) sich die Taste 20 zusammen mit den Stempelköpfen 46 plötzlich einwärts bewegt und die vorspringenden Stempelköpfe 46 durch die Aussparungen 16 des Anlageteils 14 hindurch mit den Nadelspitzen 48 in die Ohrmuschel eindringen und das auf den Nadelspitzen 48 befindliche Material sicher und zuverlässig in die Ohrmuschel injizieren.

Wird der Druck auf die Taste 20 gelöst, so bewegt sich diese unter Wirkung der Feder 22 zunächst auswärts, wodurch die Nadeln 48 zuverlässig aus der noch festgehaltenen Ohrmuschel entfernt werden. Erst anschließend öffnet sich das Zangenteil 6 und gibt die Ohrmuschel wieder frei. Wird das Zangenteil 6 weiter geöffnet, so wird der Materialträger 36 aus dem Aufnahmeteil 18 ausgeworfen.

Fig. 4 zeigt eine gegenüber Fig. 1 bis 3 etwas abgeänderte Ausführungsform der Halterung des Aufnahmeteils 18 am Gehäuse 2. Die Halterung geschieht hier mittels einer Viergelenklagerung 62. Im übrigen entspricht die Funktion der der Ausführungsform gemäß den Fig. 1 bis 3.

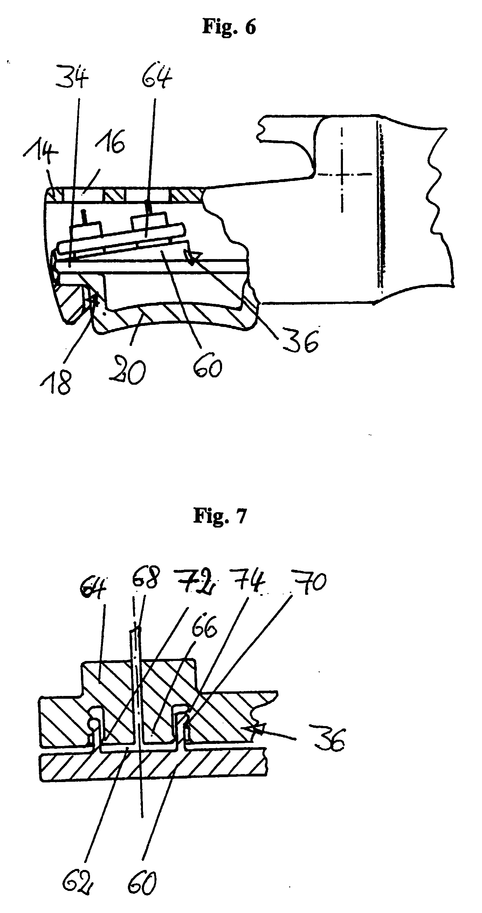

Um größere Mengen beispielsweise eines Impfstoffes injizieren zu können, ist eine Apparatur mit Kanülen anstelle von Nadeln günstiger. Eine solche Ausführungsform ist in den Fig. 6 und 7 dargestellt, die sich nur hinsichtlich des Materialträgers von der der Fig. 1 bis 3 unterscheidet. Die Tragplatte 34 des Materialträgers 36 weist hier ein Basisteil 60 auf, das mit Zylindernäpfen 62 ausgebildet ist. In diese Zylindernäpfe wird das zu injizierende Material eingefüllt. Anschließend wird ein Stempelkopf 64 mit seinen Kolben 66 und Kanülen 68 bis zu einer ersten Rastposition 70 aufgeschnappt, wobei eine Dichtlippe 72 den Zylinderraum schließt. Es versteht sich, daß die Kanülen 68 zunächst mittels eines nicht dargestellten Druckpolsters geschützt werden, das gleichzeitig überschüssiges Material aufnimmt. Nach Entfernen des Druckpolsters wird der Materialträger 36 insgesamt in das Aufnahmeteil 18 des Gerätes eingeschoben. Beim Arbeiten mit Kanülen muß der Druck zwischen Zangenteil 6 und Taste 20 länger beibehalten werden, bis das zu injizierende Material aus den Zylinderräumen durch die Kanülen in die Ohrhaut dringt. Dabei springen die die Zylindernäpfe 62 begrenzerden zylindrischen Wände in die zweite Rastposition 74, so daß eine exakt definierte Menge an zu injizierendem Material herausgepreßt wird.

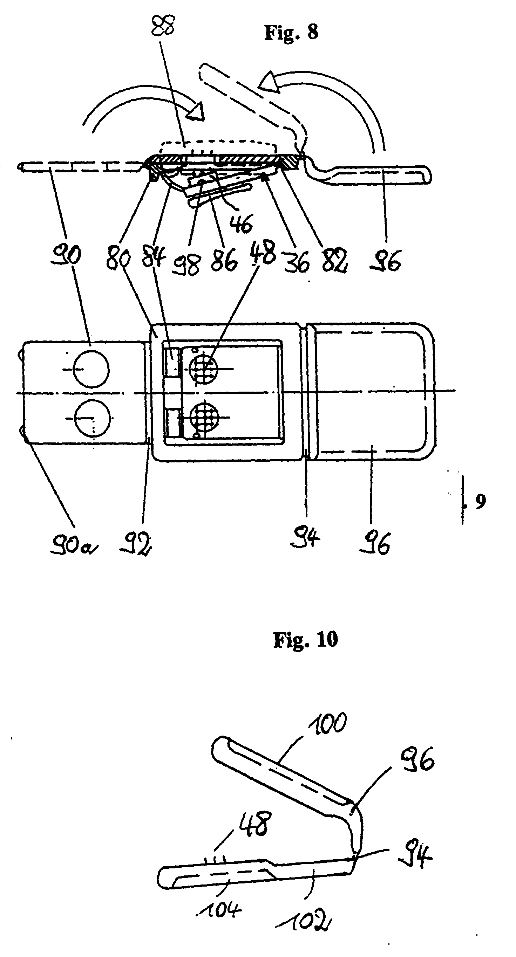

Anhand der Fig. 8 und 9 wird im folgenden eine Ausführungsposition des Gerätes beschrieben, die in ihrem Aufbau vereinfacht ist, aus wenigen Teilen besteht, und für den einmaligen Gebrauch geeignet ist.

Das Gerät weist einen Rahmen 80 auf, unter dem ein Materialträger 36 an einer Seite mittels eines Filmscharniers 82 und an der anderen Seite mit zwei angespritzten Federn 84 befestigt ist. Der Materialträger 36 trägt Stempelköpfe 46, die mit Nadelspitzen 48 bestückt sind. Es versteht sich, daß anstelle dieser Ausführungsform des Materialträgers auch eine den Fig. 6 und 7 entsprechende mit Kanülen verwendet werden kann. In diesem Fall wird eine Taste 86 vorgesehen, die die Zylindernäpfe trägt. Diese Zylindernäpfe werden vor dem Einbauen bzw. Aufschnappen der Taste 86 mit dem zu injizierenden Material bestückt. Ein Druckpolster 88 schützt die Anordnung, hält sie steril und nimmt das überschüssige Material auf.

Weiter weist die Anordnung einen Deckel 90 auf, der mittels eines Filmscharniers 92 an dem Rahmen 80 angebracht ist und, gegebenenfalls nach Entfernen des Druckpolsters 88 um 180° verschwenkbar ist und mit Rastnasen 90a im Rahmen 80 einrastet. Der Deckel dient als dem Anlageteil 14 entsprechendes Anlageteil.

Auf der anderen Seite des Rahmens 80 ist mittels eines Filmscharniers ein dem Zangenteil 6 entsprechendes Zangenteil 96 angebracht.

Die Funktion des Gerätes entspricht der des vorbeschriebenen Gerätes:

Beim Schließen des Zangenteils 96 wird die Ohrmuschel zwischen dem Zangenteil 96 und dem Deckel 90 aufgenommen und flachgedrückt. Bei Erhöhen des Druckes schnappen die Federn 84 um, so daß sich der Materialträger 36 ruckartig einwärts bewegt und die Nadeln in die Ohrhaut vorschnellen. Ein am Materialträger 36 ausgebildeter Anschlag 98 begrenzt diese Bewegung. Wenn der von der Hand ausgeübte Druck gelöst wird, fährt der Materialträger 36 zurück. Anschließend öffnet sich das Zangenteil, so daß das Gerät von der Ohrmuschel entfernt und entsorgt werden kann.

Es versteht sich, das vielfältige Abänderungen der beschriebenen Ausführungsformen möglich sind. Beispielsweise kann das Gerät gemäß Fig. 1 bis 3 mittels eines integrierten Elektromotors der aus Batterien gespeist wird, betrieben werden. Das Gerät kann dann lediglich an dem gemäß Fig. 1 rechten Abschnitt des Gehäuses gehalten werden und wird über einen dort angebrachten Mikroschalter in Betrieb gesetzt. Innerhalb des Gehäuses ist ausreichend Platz für eine Batterie und eines Antriebs.

Eine besonders einfache Ausführungsform des Gerätes ist in Fig. 10 dargestellt. Das Zangenteil 96 der Fig. 8, das lediglich als Rahmen ausgebildet ist oder als flächiges Teil mit einer Handhabungsvertiefung 100, ist über ein Filmscharnier 94 mit einem Anlageteil 102 verbunden, das ebenfalls eine Handhabungsvertiefung 104 aufweist. Das Anlageteil 102 trägt unmittelbar starr die Nadelspitzen 48 zum Injizieren des Materials. Die Eindringtiefe der Nadelspitzen 48 in die Ohrmuschel ist durch das Maß gegeben, um das die Nadelspitzen 48 aus dem Anlageteil 102 vorstehen. Zum Impfen wird die Ohrmuschel zwischen die Teile 100 und 102 genommen. Die Teile werden zusammengedrückt.

Beim Einklemmen der Ohrmuschel dringen die mit Impfmaterial beschichteten Nadelspitzen 48 in die Ohrmuschel ein. Das Öffnen muß sorgfältig geschehen, da das Anlageteil 102 erst seitlich zur Ohrmuschel bewegt werden darf, wenn sich die Nadelspitzen 48 vom Ohr entfernt haben.

Es versteht sich, daß zur Verstärkung der Öffnungskraft des Gerätes gemäß Fig. 10 das Filmscharnier 94 durch eine Feder ergänzt werden kann.

## Patentansprüche

1. Impfverfahren, bei dem ein Impfstoff von außen in ein Körpergewebe injiziert wird, dadurch gekennzeichnet, daß der Impfstoff in eine Ohrmuschel injiziert wird.

2. Impfverfahren nach Anspruch 1, dadurch gekennzeichnet, daß der Impfstoff DNA und/oder RNA enthält.

3. Verwendung eines Impfstoffs, dadurch gekennzeichnet, daß der Impfstoff in eine Ohrmuschel injiziert wird.

4. Verwendung eines Impfstoffs nach Anspruch 3, dadurch gekennzeichnet, daß der Impfstoff DNA und/oder RNA enthält.

5. Gerät zum Injizieren eines Materials, insbesondere Impfstoffs, in eine Ohrmuschel, enthaltend
eine Klemmvorrichtung (14, 6; 90, 96; 102) zum Einklemmen eines Bereiches der Ohrmuschel und
eine Injektionsvorrichtung (36, 46, 48; 68), die zum Injizieren des Materials in die Ohrmuschel eindringt.

6. Gerät nach Anspruch 5, wobei die Injektionsvorrichtung (48) starr an einem Teil der Klemmvorrichtung (96, 102) befestigt ist.

7. Gerät nach Anspruch 5, wobei die Injektionsvorrichtung (36, 46, 48; 68) bei in der Klemmvorrichtung (14, 6; 90, 96) eingeklemmter Ohrmuschel auf das Ohr zubewegbar ist.

8. Gerät nach Anspruch 7, wobei die Klemmvorrichtung ein Anlageteil (14; 90) und ein bewegliches Zangenteil (6; 96) enthält, die zwischen sich einen Zwischenraum bilden, in den eine Ohrmuschel einführbar ist, und die Injektionsvorrichtung einen auf der vom Zangenteil abgewandten Seite des Anlageteils angeordneten, gegen die Kraft einer Federeinrichtung (22; 24; 84) zum Anlageteil hin bewegbaren Materialträger (36), mit wenigstens einem Vorsprung (46, 48; 68) enthält,
wobei beim Schließen des Zangenteils durch Aufbringen einer zwischen der von dem Anlageteil abgewandten Rückseite des Materialträgers und der Außenseite des Zangenteils wirksamen Kraft zunächst die in den Zwischenraum eingeführte Ohrmuschel in flächige Anlage an das Anlageteil (14; 90) gelangt und anschließend der wenigstens eine an dem Materialträger (36) ausgebildete Vorsprung (46, 48; 68) mit seinem freien Ende durch das Anlageteil (14; 90) hindurch zum Injizieren des an dem Vorsprung befindlichen Materials in die Ohrmuschel eindringt.

9. Gerät nach Anspruch 8, wobei die den Materialträger (36) abstützende Federeinrichtung (22, 24; 84) zu Beginn ihrer Verformung eine große und dann eine kleinere Federkraft aufweist.

10. Gerät nach Anspruch 8 oder 9, wobei der wenigstens eine, an dem Materialträger (36) ausgebildete Vorsprung als Stempelkopf (46) ausgebildet ist, der wenigstens eine mit dem zu injizierenden Material versehene Nadelspitze (48) trägt.

11. Gerät nach Anspruch 8 oder 9, wobei der Materialträger (36) ein Basisteil (60) und einen relativ zu dem Basisteil verschiebbaren Stempelkopf (64) aufweist, von dem aus eine Kanüle (68) vorsteht, wobei der Stempelkopf beim Schließen des Zangenteils (6) in Anlage an das Anlageteil (14) kommt, wodurch es sich unter Verminderung des Volumens einer das zu injizierende Material enthaltenden Kammer (62) und Ausspritzen des Materials aus der mit der Kammer verbundenen Kanüle in Richtung auf das Basisteil (60) bewegt.

12. Gerät nach Anspruch 11, wobei in der Verschiebbarkeit zwischen Basisteil (60) und Stempelkopf (64) zwei Rastpositionen (70, 74) vorgesehen sind.

13. Gerät nach einem der Ansprüche 8 bis 12, wobei Anschläge (52; 54; 98) ausgebildet sind, mit denen die Eindringtiefe des wenigstens einen Vorsprungs in die Ohrmuschel einstellbar ist.

14. Gerät nach einem der Ansprüche 8 bis 13, wobei der Materialträger (36) in ein Aufnahmeteil (18) einschiebbar ist, welches mit einem Tastenbereich ausgebildet ist und beweglich an einem Gehäuse (2) gehalten ist, das auch das Zangenteil (6) hält.

15. Gerät nach Anspruch 14, wobei das Zangenteil (6) gemeinsam mit dem Aufnahmeteil (18) auf einem Bolzen (4) schwenkbar gelagert ist.

16. Gerät nach Anspruch 14 oder 15, wobei das Zangenteil (6) einen Auswerfernocken (12) aufweist, welcher bei Öffnen des Zangenteils den Materialträger (36) aus dem Aufnahmeteil (18) ausschiebt.

17. Gerät nach einem der Ansprüche 8 bis 13, wobei das Zangenteil (96) und der Materialträger (36) als aus Kunststoff bestehendes Spritzgußteil einteilig mit einem Rahmen (80) ausgebildet sind, an dem sie mittels Filmscharnieren (82, 94) befestigt sind, und die Federeinrichtung (84) an das Rahmenteil und/oder den Materialträger angespritzt ist.

18. Gerät nach Anspruch 17, wobei an der vom Zangenteil (96) abgewandten Seite des Rahmens (80) mittels eines Filmscharniers (92) ein Deckel (90) angebracht ist, welcher in seinem auf den Rahmen geklappten Zustand das Anlageteil bildet.
